(19) Europäisches Patentamt
European Patent Office
Office européen des brevets

(11) EP 2 987 486 A1

(12) **EUROPEAN PATENT APPLICATION**
published in accordance with Art. 153(4) EPC

(43) Date of publication:
**24.02.2016 Bulletin 2016/08**

(21) Application number: **14784805.5**

(22) Date of filing: **02.04.2014**

(51) Int Cl.:
***A61K 31/352*** *(2006.01)* ***A61K 47/10*** *(2006.01)*
***A61P 35/02*** *(2006.01)* ***A61P 43/00*** *(2006.01)*

(86) International application number:
**PCT/JP2014/059739**

(87) International publication number:
**WO 2014/171333 (23.10.2014 Gazette 2014/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.04.2013 JP 2013086657**

(71) Applicant: **Flora Co. Ltd.**
**Mie 510-0855 (JP)**

(72) Inventors:
- **KAWASE, Yoshinari**
**Inabe-shi**
**Mie 511-0404 (JP)**
- **KAWASE, Yoshiyasu**
**Inabe-shi**
**Mie 511-0404 (JP)**
- **FUJII, Tokio**
**Yokkaichi-shi**
**Mie 510-0855 (JP)**
- **KOBAYASHI, Masaki**
**Nissin-shi**
**Aichi 470-0105 (JP)**

(74) Representative: **Grünecker Patent- und Rechtsanwälte**
**PartG mbB**
**Leopoldstraße 4**
**80802 München (DE)**

(54) **MITOCHONDRIA ACTIVATOR**

(57) In order to allow differentiation induction of cancer cells into normal cells, the present invention provides an activator of mitochondria containing a component expected to have an epigenetic action, or provides a cell differentiation inducer having a novel composition that has an action to activate mitochondria. The activator of mitochondria contains, as an effective component, 0.03 to 300 μg/mL of a single component of kaempferol, or a mixture component of kaempferol and glycerol. These agents can promote DNA methylation, activate resting genes in cancer cells such as leukemia cells to promote their normal cell division according to the normal cell cycle, and activate mitochondrial functions, to thereby allow continuous differentiation induction into normal cells without arresting the cell differentiation.

EP 2 987 486 A1

**Description**

TECHNICAL FIELD

The present invention relates to an activator of mitochondria which enhances physiological activities of mitochondria and increases the abundance of mitochondria in cells to promote normal division and differentiation of the cells, to thereby allow differentiation induction of leukemia cells and the like into normal cells.

BACKGROUND ART

In general, mitochondria activate cellular functions and promote, especially in muscle cells, energy consumption. Known examples of activators for their functions include benzimidazole derivatives, and they are known to have, for example, effects to prevent obesity and diabetes (Patent Document 1 listed below).
It is not widely known that such activators of mitochondria have roles to promote normal division and differentiation of cells containing mitochondria.
For example, in a mechanism of development of acute promyelocytic leukemia, differentiation inhibition occurs in granulocytes and the like, and the cells whose differentiation is inhibited are found to have low mitochondrial activity. However, the causal relationship between the differentiation inhibition and the mitochondrial activity is not well known.
There are cancer growth inhibitors containing generally known flavonols, which are prepared using extracts of fruits and the like as materials. A known example of such cancer growth inhibitors is a cancer growth inhibitor containing components such as quercetin and kaempferol (Patent Document 2 listed below).
It is also known that kaempferol, which is known as a polyphenol contained in legume plants, can be used for functional foods, cosmetics, reagents, and pharmaceuticals, after its extraction from the aqueous fraction (Patent Document 3 listed below).
A cell differentiation inducer discovered by the present inventors which is derived from natural components and shows differentiation induction activity for cancer cells of leukemia, skin cancer, and the like has been disclosed, and is described in a publication (Patent Document 4 listed below) as a cell differentiation inducer against leukemia cells containing as an effective component an ethyl alcohol-soluble component of a dried product of a water-soluble mixed extract prepared by mixing the following hot water extracts at controlled ratios: hot water extracts obtained by adding water to ground wood products of Japanese cedar, Japanese cypress, and pine, boiling the resulting mixtures, and filtering the boiled products to remove the resin content and the solid content therefrom; and a hot water extract obtained by adding psyllium water, boiling the resultant, and filtering the boiled product.

PRIOR ART DOCUMENTS

[Patent Documents]

[Patent Document 1] JP 2004-67629 A
[Patent Document 2] JP 2012-25724 A
[Patent Document 3] JP 2007-284369 A
[Patent Document 4] JP 4717362 B

SUMMARY OF THE INVENTION

PROBLEMS TO BE SOLVED BY THE INVENTION

However, the effective components of the conventional cancer growth inhibitors and cell differentiation inducers described above are merely a large number of unspecified components contained in the extracts derived from the natural products, and identification of particular effective components has not been done. Thus, identification of the effective components remained to be done for increasing reliability of the growth inhibitory action on cancer cells, in order to allow more efficient exertion of the expected actions.
Kaempferol, as a flavonoid, is known to have a cancer growth inhibitory effect through an action to eliminate free radicals. However, kaempferol has not been shown to be an effective component having a cell differentiation-inducing ability.
As an effective component of therapeutic agents for acute preleukemia, retinoic acid, which is a vitamin A derivative, is known. In cases of its administration for leukemia cells, the ratio at which myelocytes differentiate into

neutrophils is not sufficiently high, and the ratio at which myelocytes differentiate into monocytes, whose phagocytosis is weaker than that of neutrophils, is high. Thus, the effect to allow phagocytosis of cancer cells for exertion of anticancer properties is not sufficiently produced, which is problematic.

Accordingly, an object of the present invention is to solve the above-described problems by providing an activator of mitochondria containing, among components derived from natural products contained in fruits and the like, a component expected to have an epigenetic action that allows differentiation induction of cancer cells, especially cancer cells of human and the like, into normal cells, or providing a cell differentiation inducer having a novel composition that has an action to activate mitochondria.

Another object of the present invention is to provide a cell differentiation inducer containing an effective component having a cell differentiation-inducing capacity which, upon administration for human leukemia cells, increases the ratio at which myelocytes differentiate into neutrophils, and decreases the ratio at which myelocytes differentiate into monocytes, whose phagocytosis is weaker than that of neutrophils, thereby enabling efficient phagocytosis of cancer cells and sufficient enhancement of the performance to exert anticancer properties.

## MEANS FOR SOLVING THE PROBLEMS

In order to solve the problems described above, the present invention provides an activator of mitochondria comprising, as an effective component, a single component of kaempferol, or a mixture component of the combination of kaempferol and glycerol.

Since the activator of mitochondria of the present invention constituted as described above contains as an effective component the single component of kaempferol, or the mixture component of the combination of kaempferol and glycerol, the activator of mitochondria of the present invention has an epigenetic action that promotes the so called DNA methylation which causes transfer of a methyl group to cytosine in mitochondrial DNA of human leukemia cells and the like to change the cytosine to 5-methylcytosine.

The DNA methylation described above is indispensable for normal development, and involved in many key steps such as gene imprinting, inactivation of the X chromosome, suppression of repetitive factors, and carcinogenesis.

Such an action activates resting genes in cancer cells such as leukemia cells, to promote their normal cell division according to the normal cell cycle. That is, the activator of mitochondria of the present invention activates mitochondrial functions to allow continuous induction of differentiation of the cells into normal cells, without arresting their differentiation.

For sufficient exertion of the above-described action, the concentration of the effective component in the activator of mitochondria is preferably 0.03 to 300 $\mu$g/mL.

This is because, at a concentration of as low as less than 0.03 $\mu$g/mL (=30 ng/mL), the activity and the growth ability of mitochondria are inhibited, while at a concentration of as high as more than 300 $\mu$g/mL, no remarkable increase in the effect can be expected, so that such a concentration is impractical from the viewpoint of efficiency.

In cases where a mixture component of the combination of kaempferol and glycerol is used as an effective component in the present invention, the mixing ratio by mass between kaempferol and glycerol in the activator of mitochondria is preferably within the range of 99.5:0.5 to 0.5:99.5.

In cases where kaempferol and glycerol are used in combination, and the mixing ratio of glycerol is as low as less than 0.5% by mass, the activity and the growth ability of mitochondria may be instable, which is not preferred. In cases where kaempferol and glycerol are used in combination, and the mixing ratio of glycerol is as high as more than 99.5% by mass, the activity and the growth ability of mitochondria can be found, but the amount of the other part of the effective component, kaempferol, needs to be appropriately controlled depending on the purpose of use of the activator of mitochondria.

An especially preferred use of the activator of mitochondria is an activator of mitochondria for acute promyelocytic leukemia cells. A cell differentiation inducer for acute promyelocytic leukemia cells, containing as an effective component the activator of mitochondria described above may also be provided.

## EFFECT OF THE INVENTION

Since the present invention provides an activator of mitochondria containing, as an effective component, a single component of kaempferol, or a mixture component of the combination of kaempferol and glycerol, the activator of mitochondria can be advantageously provided as an activator of mitochondria containing a component expected to have an epigenetic action that allows differentiation induction of cancer cells, especially cancer cells of human and the like, into normal cells, or as a cell differentiation inducer having a novel composition that has an action to activate mitochondria.

The cell differentiation inducer for acute promyelocytic leukemia cells (HL60) containing as an effective component the activator of mitochondria of the present invention remarkably increases the ratio at which myelocytes differentiate into neutrophils, and decreases the ratio at which myelocytes differentiate into monocytes, whose phagocytosis is weaker than that of neutrophils. Thus, the cell differentiation inducer has an advantage that the ratio of neutrophils, which efficiently phagocytose cancer cells, can be increased to allow exertion of anticancer properties.

BRIEF DESCRIPTION OF THE DRAWINGS

Fig. 1 is a chart showing the result of a cytotoxicity test for investigation of mitochondrial activation, wherein the relationship between the amount of the effective component (kaempferol, or kaempferol and glycerol) added and the absorbance (viable cell count) is shown.

Fig. 2 is a chart showing the relationship between the amount of kaempferol added and the dead cell ratio after 5 days or 10 days of culture.

Fig. 3 is a chart showing the capacity to induce differentiation into neutrophils by 5 days of culture, wherein the relationship between the amount of the reagent (ethyl alcohol, vitamin $D_3$, or retinoic acid) or the effective component (kaempferol and glycerol, or kaempferol) added and the ratio of NBT-stained cells is shown.

Fig. 4 is a chart showing the capacity to induce differentiation into neutrophils by 10 days of culture, wherein the relationship between the amount of the reagent (ethyl alcohol, vitamin $D_3$, or retinoic acid) or the effective component (kaempferol and glycerol, or kaempferol) added and the ratio of NBT-stained cells is shown.

Fig. 5 is a chart showing the capacity to induce differentiation into monocytes by 5 days of culture, wherein the relationship between the amount of the reagent (ethyl alcohol, vitamin $D_3$, or retinoic acid) or the effective component (kaempferol and glycerol, or kaempferol) added and the ratio of esterase-stained cells is shown.

Fig. 6 is a chart showing the capacity to induce differentiation into monocytes by 10 days of culture, wherein the relationship between the amount of the reagent (ethyl alcohol, vitamin $D_3$, or retinoic acid) or the effective component (kaempferol and glycerol, or kaempferol) added and the ratio of esterase-stained cells is shown.

Fig. 7 is a chart showing the capacity to induce differentiation by 10 days of culture, wherein the relationship between the amount of the reagent (ethyl alcohol, vitamin D3, or retinoic acid) or kaempferol added and the ratio of differentiated cells or the amount of methylated DNA is shown.

Fig. 8 is a chart showing the result of a test in which viable cells were counted by trypan blue staining.

Fig. 9 is a chart showing the result of a test for measurement of the mitochondrial activity (maximum reaction rate).

Fig. 10 is a chart showing the result of a test for measurement of the mitochondrial activity (unit value).

MODE FOR CARRYING OUT THE INVENTION

The activator of mitochondria of the present invention contains, as an effective component, a single component of kaempferol, or a mixture component of the combination of kaempferol and glycerol, and exerts an epigenetic DNA methylation action which causes transfer of a methyl group to cytosine in mitochondrial DNA of human leukemia cells and the like to change the cytosine to 5-methylcytosine.

That is, the cell differentiation inducer of the present invention has an action to induce cell differentiation of undifferentiated cells such as cancer cells, and has a potential to induce normal differentiation of cells involved in diseases that develop due to abnormal DNA methylation, histone deacetylation, or the like, such as acute promyelocytic leukemia, colon cancer, prostate cancer, and diabetic nephropathy.

The kaempferol used in the present invention is a naturally occurring flavonol with a molecular formula of $C_{15}H_{10}O_6$ and a molecular weight of 286.24, represented by Chemical Formula 1 below. Naturally derived kaempferol are contained in leaves of geranium herb and ejis herb, cabbage, beans, tomato, and the like, in the forms of glycosides in which a sugar(s) is/are bound to 3-position, or to 3-position and 7-position.

[Chemical Formula 1]

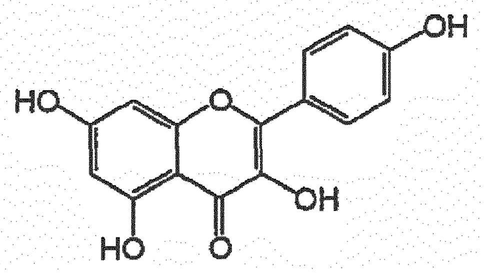

It has been revealed that kaempferol and glycerol are also contained in a cell differentiation inducer which is derived from natural components and shows differentiation induction activity for cancer cells of leukemia, skin cancer, and the like (see Patent Document 4). These naturally-derived components are contained in an ethyl alcohol-soluble component of a dried product of a water-soluble mixed extract prepared by mixing the following hot water extracts at controlled ratios: hot water extracts obtained by adding water to ground wood products of Japanese cedar, Japanese cypress, and pine, boiling the resulting mixtures, and filtering the boiled products to remove the resin content and the solid content therefrom; and a hot water extract obtained by adding water to psyllium, boiling the resulting mixture, and filtering the boiled product.

Although the glycerol used in the present invention is a trihydric alcohol represented by the rational formula $C_3H_5(OH)_3$ or the molecular formula $C_3H_8O_3$, oligoglycerols such as diglycerol, and polyglycerols, may be used individually or as a mixture of two or more of these depending on the mode of the formulation.

The effective component of the activator of mitochondria of the present invention, or the cell differentiation inducer for acute promyelocytic leukemia cells containing the activator of mitochondria as an effective component, is highly safe since the component is contained in foods such as the vegetables described above, and safety of glycerol is well known.

Examples of the dosage form include oral formulations such as powders, fine granules, granules, tablets, coated tablets, and capsules; injectable formulations; and external preparations. The formulations can be produced by normal methods using normal formulation carriers.

That is, for production of an oral formulation, the effective component and a vehicle, and, if necessary, a binder, disintegrator, lubricant, coloring agent, corrective, and/or the like are mixed, and the resulting mixture is formulated into the dosage form described above by a conventional method.

Examples of the vehicle include lactose, corn starch, glucose, sorbitol, crystalline cellulose, and silicon dioxide. Examples of the binder include polyvinyl alcohol, methyl(ethyl)cellulose, gum arabic, tragacanth, gelatin, and polyvinyl pyrrolidone. Example of the disintegrator include starch, agar, gelatin, crystalline cellulose, calcium carbonate, dextrin, and pectin. The tablets and the granules may have a sugar coating, or may be have another type of coating, if necessary.

In cases where an injectable formulation is produced, one or more of pH adjusters, resolvents, isotonic agents, and the like, and, if necessary, solubilizers, stabilizers, and the like is/are added to the effective component, and the resulting mixture is subjected to formulation by a conventional method.

The method for producing an external preparation is not limited, and the external preparation may be produced by a conventional method. That is, examples of its base material include animal and vegetable oils, ester oil, waxes, higher alcohols, fatty acids, silicon oil, surfactants, phospholipids, clay minerals, and purified water. One or more of pH adjusters, antioxidants, chelating agents, and the like may be further added, if necessary.

In addition, if necessary, one or more of components such as blood flow promoters, microbicides, anti-inflammatory agents, cell activators, vitamins, amino acids, moisturizers, and keratolytic agents may be added. The base material is added such that a concentration normally set for production of an external preparation is achieved.

The clinical dose of the effective component in the present invention is not limited, and varies depending on the symptom, severity, age, complication, and the like, and also on the type and the administration route of the compound. The concentration of the effective component for cancer cells and the like is 0.03 to 300 μg/mL, preferably 30 to 300 μg/mL.

EXAMPLES

Each of the activator of mitochondria of Example 1, which contains kaempferol as an effective component, and the activator of mitochondria of Example 2, which contains 1 part by mass of kaempferol and 9 parts by mass of glycerol, was mixed with distilled water (SDW) as a base material at a predetermined concentration.

That is, as shown in Figs. 1 to 7, the content of each of these activators of mitochondria was $3.2\times10^{-4}$% by

mass, $1.0\times10^{-3}$% by mass, $3.2\times10^{-3}$% by mass, 0.01% by mass, 0.032% by mass, 0.1% by mass (30 ng/mL), 0.32% by mass, 1% by mass, 3.2% by mass, or 10% by mass (=300 μg/mL).

These activators of mitochondria (Examples 1 and 2) were applied to cells cultured by the following method, to perform a cytotoxicity test (mitochondrial activity level [counting of viable cells]), cell differentiation induction test, esterase staining test, and NBT reduction staining test. The results are shown in Figs. 1 to 7.

The methods for these tests were as described below.

1) Conditions for the cell culture

HL60 cells were cultured using a test medium (RPMI 1640 medium, 10% FBS, antibiotic-free) to the logarithmic growth phase in a CO: incubator (5% Cq, 37°C).

2) Cytotoxicity test (evaluation of the toxicity based on the viability, that is, the survival rate or the death rate, of cells)

To each well of a 96-well plate, 0.1 mL of HL6O cells were plated at a concentration of $5\times10^4$ cells/mL, and a test substance (7 serial dilutions: a 10-fold dilution series ranging from 10%; the test substance was preliminarily sterilized by filtration) was added to the well at the same time. After 3 days of the culture, a viable cell assay reagent SF (final concentration, 10%) was added to the cell suspension culture liquid, and viable cells were counted. The test was carried out in 3 replicates (n=3). The viability of the cells was evaluated using a viable cell counting reagent SF (WST-8) based on measurement of the absorbance.

3) Cell differentiation induction test

To each well of a 6-well plate, 1 mL of HL60 cells were plated at a concentration of $5\times10^4$ cells/mL, and a test substance at five serial concentrations (a 10-fold dilution series ranging from 10%) was added to the well at the same time as the plating. The cells in the plate were cultured for 5 days or 10 days under 5% Cq at 37°C. The cell suspension in each well was recovered by centrifugation, and the number of cells, the NBT reducing capacity, and the esterase activity of the recovered cells were measured. In addition, changes in the karyotype of each cell was observed by Carrazzi's hematoxylin staining. The ratio of stained cells in each sample was measured to evaluate the differentiation induction capacity. The test was carried out in 5 replicates (n=5). A sample without addition of the test substance, and 0.1 % ethyl alcohol (solvent control for the positive control) were used as negative controls, and retinoic acid (dissolved in ethyl alcohol; final concentration, 1 μM) and active vitamin $D_3$ (dissolved in ethyl alcohol; final concentration, 10 nM) were used as positive controls. Details of each staining method are described below.

3-1) Esterase staining and Carrazzi's hematoxylin staining

Esterase staining was carried out as follows using an esterase staining kit (manufactured by Muto Pure Chemicals Co., Ltd.). The concentration of the recovered cells was adjusted to $1\times10^6$ cells/mL with PBS. The resulting cell solution was thinly spread on a slide glass, and left to stand for 10 minutes. Subsequently, a fixation solution was added dropwise onto the cells in a refrigerator, and the sample was then left to stand for 30 seconds, followed by washing with running water for 30 seconds. Thereafter, an esterase reaction solution was added dropwise to the sample, and the sample was then left to stand at 37°C (in a humidity chamber) for 30 minutes, followed by washing with running water for 30 seconds. Carrazzi's hematoxylin solution was added dropwise to the sample, and the sample was then left to stand at room temperature for 10 minutes, followed by washing with running water for 5 minutes. After drying the sample, 50% glycerol solution was added dropwise to the sample, and the sample was then embedded with a cover glass, followed by counting cells stained reddish brown or blue under the microscope.

3-2) NBT reduction staining

The concentration of the recovered cells was adjusted to $1\times10^6$ cells/mL with RPMI 1640 medium (SIGMA, R8758, serum-free), and an equal volume of NBT solution (PBS supplemented with 0.2% NBT and 20% FBS, which was used after filtration) was added thereto. TPA was added to the resulting mixture to a final concentration of 6 μM, and the mixture was then stirred. Thereafter, the mixture was left to stand at 37°C for 30 minutes, and centrifuged at 1000 rpm for 5 minutes, followed by discarding the supernatant. Subsequently, the cells were resuspended in PBS, and applied onto a slide glass, followed by counting cells stained blue under the microscope.

The results of the tests are shown in Figs. 1 to 7.

Fig. 1 is a diagram showing the mitochondrial activities observed with the various concentrations of kaempferol or the mixture of kaempferol and glycerol. In the group in which the mixture of kaempferol and glycerol was added, the

mitochondrial activity tended to increase when the concentration of the mixture was within the range of $3.2\times10^{-4}$% to 0.032%. The mitochondrial activity did not increase at the higher concentrations of up to 10% (300 μg).

On the other hand, in the group of the various concentrations of kaempferol, the mitochondrial activity was suppressed when the concentration of kaempferol was within the range of $3.2\times10^{-4}$% to 0.032%, and the mitochondrial activity increased when the concentration of kaempferol was within the range of 0.1% (30 ng/mL=0.03 μg/mL) to 10% (300 μg/mL).

As shown in Fig. 2, the dead cell count according to trypan blue staining after 5 days of the culture was 2% to 4%, and no difference depending on the concentration was found. Thus, it can be seen that use of kaempferol alone is preferred for activation of mitochondria.

As shown in Figs. 3 and 4, in the test for induction of differentiation into neutrophils (positive for NBT staining) by 5 days or 10 days of culture, the stained cells increased dependently on the concentration of kaempferol/glycerol. This indicates that kaempferol has an action to activate mitochondria and a differentiation induction action, and that kaempferol/glycerol has a favorable differentiation induction action.

As shown in Fig. 3, the kaempferol group and the kaempferol/glycerol group after 5 days of the culture showed concentration-dependent improvement of the differentiation ratio, and 300 μg of kaempferol/glycerol showed a differentiation ratio which is slightly more than twice the differentiation ratio observed with 300 μg of kaempferol.

As shown in Fig. 4, based on the ratio of NBT-stained cells after 10 days of the culture, the differentiation ratios in the kaempferol group were decreased on Day 10 of the culture. The differentiation ratio was 3% even with 300 μg of kaempferol. In contrast, the kaempferol/glycerol group showed improved differentiation rates of 4%, 8%, 7%, 9%, and 14% at the concentrations of kaempferol/glycerol of 30 ng, 0.001%, 0.1%, 1%, and 10%, respectively. In the case of addition of retinoic acid, the differentiation ratio was 68%. In the case of addition of active vitamin $D_3$, the differentiation ratio was decreased by half to 4%.

From these results, it can be seen that, by the use of the single kaempferol component, or the mixture component of the combination of kaempferol and glycerol as an effective component, the number of granulocytes such as neutrophils significantly increased dependently on the concentration with time during the culture period between Day 5 and Day 10.

As shown in Figs. 5 and 6, in the esterase staining for evaluation of differentiation into monocytes, no significant difference was found among the different concentrations or the different culture periods in each group. In the test using retinoic acid, the differentiation was found at a ratio of 2% on Day 5 of the culture, and 14% on Day 10 of the culture. Kaempferol/glycerol did not show such a tendency.

Thus, it is thought that differentiation due to kaempferol/glycerol occurs at an earlier stage than differentiation due to retinoic acid. That is, in the cases where the single kaempferol component, or the mixture component of the combination of kaempferol and glycerol as an effective component was administered for human leukemia cells, the ratio at which myelocytes differentiated into neutrophils was high, while the ratio at which myelocytes differentiated into monocytes, whose phagocytosis is weaker than that of neutrophils, was low.

It can therefore be seen that, by use of the single kaempferol component, or the mixture component of the combination of kaempferol and glycerol as an effective component, a cell differentiation inducer which enables efficient phagocytosis of cancer cells and sufficient enhancement of the performance to exert anticancer properties can be provided.

As shown in Fig. 7, as a result of addition of various concentrations of kaempferol, it was found that the DNA methylation level in the HL60 cells increased at a kaempferol concentration of 0.001%, and decreased at a kaempferol concentration of 10% (300 μg/mL).

From these results, it was found that kaempferol can be used as a demethylation and apoptotic agent having a better differentiation induction capacity than retinoic acid, which is a differentiation inducer, and can also be used for preventing aging of cells having decreased methylase activity, that is, as a novel anti-aging agent which is a differentiation inducer (metabolism activator) for cells in a precancer state or for fat cells including a large amount of methylated cells.

[Examples 3 and 4]

A mixture of 1 part by mass of kaempferol and 200 parts by mass of glycerol was prepared as Example 3, and a composition composed of components extracted from a kaempferol-containing plant using ethyl alcohol and acetonitrile, wherein $3.0\times10^{-4}$% by mass kaempferol and 99.9997% by mass glycerol are contained, was provided as Example 4.

Using Examples 3 and 4 as test substances, a "viable cell counting and mitochondrial activity measurement test" was carried out as follows to study whether the action of the activator of mitochondria is based on differentiation of HL60 cells and an increase in the number of the cells, which results in an increase in the amount of mitochondria to cause apoptosis, or based on induction of differentiation of HL60 cells without an increase in the number of cells, wherein activation of mitochondrial functions occurs in each cell to cause apoptosis.

[Viable Cell Counting and Mitochondrial Activity Measurement Test]

In each of T25 flasks, 5 mL of HL60 cells were seeded at $1\times10^7$ cells/flask. At the same time as the seeding, test substances (ethyl alcohol, retinoic acid, dimethyl sulfoxide, rotenone, SDW, and Examples 3 and 4) were fed to the flasks.

The addition of Example 3 to the medium was carried out after its sterilization by filtration. In Example 4, 10 mL of the test substance was warmed at 60°C to evaporate the whole solvent, and 10 mL of sterile water was newly added thereto to dissolve the resultant. The resulting solution was sterilized by filtration, and then added to the medium. The retinoic acid to be added was provided as a concentrated solution containing retinoic acid at a concentration of 100 mM in ethyl alcohol, and the rotenone to be added was provided as a concentrated solution containing rotenone at a concentration of 100 mM in DMSO.

Since Examples 3 and 4 are dissolved in a medium at a final concentration of 10%, 10% SDW (sterile distilled water) was used as their negative control (solvent control). For retinoic acid and rotenone, which are positive controls, $1.0\times10^{-4}$% ethyl alcohol and $1.0\times10^{-5}$% DMSO, respectively, were used as solvent controls.

The viable cells in the flask were cultured under 5% $CO_2$ at 37°C for 5 days. The cell suspension in each well was recovered, and subjected to counting of viable cells by trypan blue staining and measurement of the mitochondrial activity (cytochrome c oxidase activity). Each assay was carried out in 5 replicates. The measurement methods were as follows.

<Counting of Viable Cells by Trypan Blue Staining>

To 50 μL of a cell suspension culture liquid, 50 μL of a trypan blue solution was added, and the reaction was allowed to proceed for several minutes. Under the microscope, unstained cells and stained cells were counted using a hemocytometer. The number of dead cells (stained cells) per total number of cells was determined to calculate the number of live cells (cells/mL) and the dead cell ratio (%). The results are shown in Fig. 8.

<Measurement of Mitochondrial Activity>

1. Extraction of Mitochondrial Fraction

The mitochondrial fraction was extracted as follows using a Mitochondria Isolation Kit for Tissue and Cultured Cells (manufactured by BioChain Institute, Inc.) according to the instructions attached to the kit.

(1) The cell suspension after the culture was centrifuged at 600xg for 5 minutes at 4°C, and the precipitated cells were washed by adding cold 1 ×PBS in the same volume as the cell liquid. The resulting cell suspension was centrifuged again at 600×g for 5 minutes at 4°C, and the cells were recovered.

(2) To the obtained precipitate (cells), 1 mL of Mitochondria isolation buffer was added, and the cells was suspended. The resulting suspension was then transferred into a tube for a homogenizer, and the cells were homogenized with 30 to 40 strokes using a homogenizer. The homogenization was carried out on ice.

(3) The homogenate was transferred into a 2-mL tube, and centrifuged at 600xg for 10 minutes at 4°C. The precipitate (non-homogenized cells) was then removed, and the supernatant was recovered.

(4) The supernatant was centrifuged at 12,000 g for 15 minutes at 4°C, and 100 μL of Lysis Buffer was added to the precipitated mitochondria. The mitochondria was suspended, and mitochondrial proteins were eluted. The protein concentration in the mitochondrial lysate was measured by the BCA method, and adjusted to 0.1 mg/mL.

2. Measurement of Mitochondrial Activity (Cytochrome C Oxidase Activity)

The mitochondrial activity (cytochrome c oxidase activity) in the thus extracted mitochondrial lysate was calculated as follows using a mitochondrial assay kit (manufactured by BioChain Institute, Inc.) according to the instructions attached to the kit.

(1) In a cuvette, the following reaction liquid (1000 μL in total) was prepared.

| | |
|---|---|
| 1 × Enzyme assay buffer | 850 μL |
| 1 × Enzyme Dilution buffer | 50 μL |

(continued)

| | |
|---|---|
| Ferrocytochrome C substrate solution | 50 μL |
| Sample (mitochondrial lysate) | 50 μL |

(2) In the preparation of the reaction liquid, the mixture was stirred by pipetting immediately after addition of the sample (mitochondrial lysate). The resulting reaction liquid was placed in a spectrophotometer, and the absorbance (550 nm) was measured 15, 20, 30, 40, 50, 60, 90, 120, and 180 seconds thereafter.
(3) From the absorbances obtained, a time/absorbance reaction curve was prepared to determine the maximum reaction rate (ABS/min.). The specific activity (ABS/min./mg protein) was calculated from the amount of protein in the mitochondrial eluate used for the measurement. The results are shown in Fig. 9.
(4) Calculation of Unit Value
The unit value was calculated as follows according to an equation described in the instructions attached to the kit.In this equation, the following values were used: dilution=2, Vol=0.05. The results are shown in Fig. 10.

$$\text{Unit/mL} = (\Delta k \times 60 \times \text{dilution} \times 1) / \text{Vol (sample)} \times \text{In } 21.84$$

3. Significance Test

Significance tests were carried out among the comparative test groups. The tests were carried out by Student T-test, and significance was assumed at P<0.05 (null hypothesis, less than 5%). The test results were scored into three categories: P<0.05, P<0.01, and P<0.001, as shown in the figure.
Normally, NADH and NADPH produced in cells are mainly produced in mitochondria, and the viable cell count is proportional to the amount of mitochondria. In the group in which rotenone was added as a positive control as an inhibitor of the mitochondrial electron transport system complex 1, the viable cell count decreased to about half of that in the solvent control as shown by trypan blue staining.
As is evident from the results shown in Fig. 8, in the counting of viable cells by trypan blue staining, no significant increase was found in the treated groups of Examples 3 and 4.
Thus, it was thought that, in the treated groups of Examples 3 and 4, there was no apparent increase in the mitochondrial activity due to an increase in the viable cell count.
On the other hand, as is evident from the results shown in Figs. 9 and 10, in the measurement of the mitochondrial activity, the treated groups of Examples 3 and 4 showed increases in the activity to about 130% (Example 3) and 118% (Example 4) with respect to the mitochondrial activity in the solvent control. Significant differences in the mitochondrial activity were found between the group treated with retinoic acid, which is a differentiation inducer, and the test substance-treated groups.
Comparison of the mitochondrial activity between the cells before the treatment with the test substances (Day 0) and the test substance-treated groups showed that the activity significantly increased in the test substance-treated groups.
Although an influence of the solvent on the activity needs to be taken into account since the 10% SDW-treated group, which is the solvent control, showed a slight increase in the activity compared to the cells before the treatment with the test substances, the influence was very small, indicating an obvious increase in the mitochondrial activity by the treatment with each test substance.

## Claims

1. An activator of mitochondria comprising, as an effective component, a single component of kaempferol, or a mixture component of the combination of kaempferol and glycerol.

2. The activator of mitochondria according to claim 1, wherein the concentration of said effective component is 0.03 to 300 μg/mL.

3. The activator of mitochondria according to claim 1 or 2, wherein the mixing ratio by mass between kaempferol and glycerol is within the range of 99.5:0.5 to 0.5:99.5.

4. An activator of mitochondria for acute promyelocytic leukemia cells, comprising the activator of mitochondria according to any one of claims 1 to 3.

5. A cell differentiation inducer for acute promyelocytic leukemia cells, comprising as an effective component the activator of mitochondria according to any one of claims 1 to 3.

Fig. 1

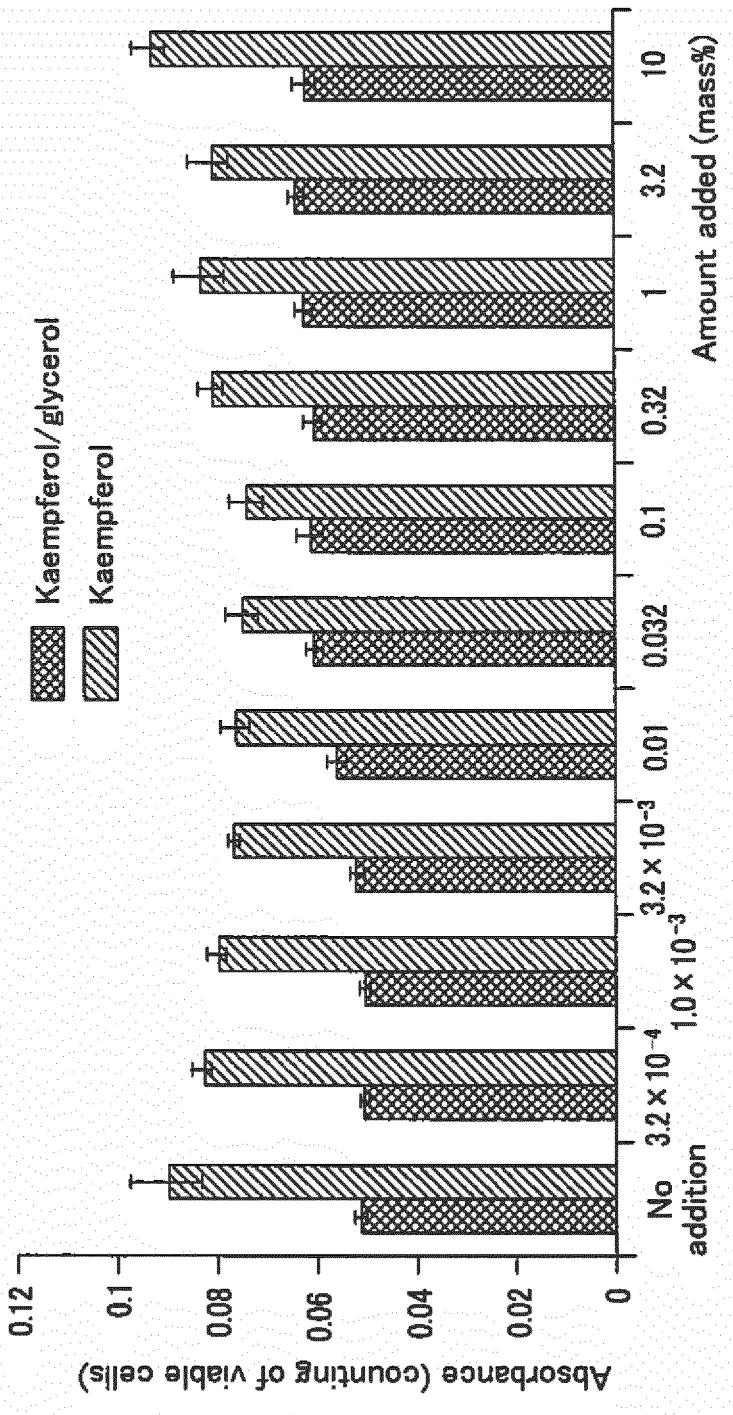
Kaempferol/glycerol
Kaempferol
Absorbance (counting of viable cells)
0.12
0.1
0.08
0.06
0.04
0.02
0
No addition
3.2×10⁻⁴
1.0×10⁻³
3.2×10⁻³
0.01
0.032
0.1
0.32
1
3.2
10
Amount added (mass%)

Fig.2

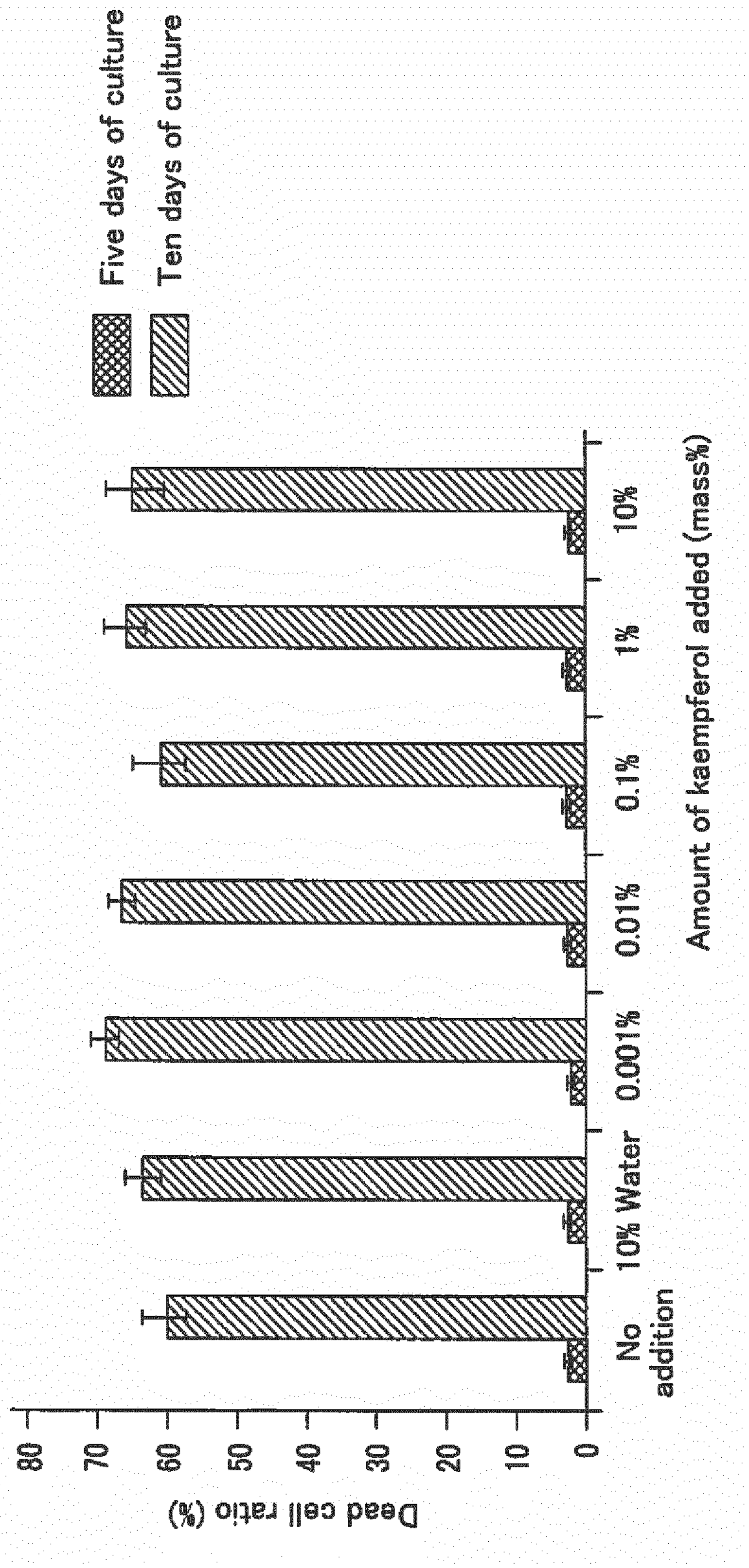
Five days of culture
Ten days of culture
Dead cell ratio (%)
80
70
60
50
40
30
20
10
0
No addition
10% Water
0.001%
0.01%
0.1%
1%
10%
Amount of kaempferol added (mass%)

Fig.3

Five days of culture

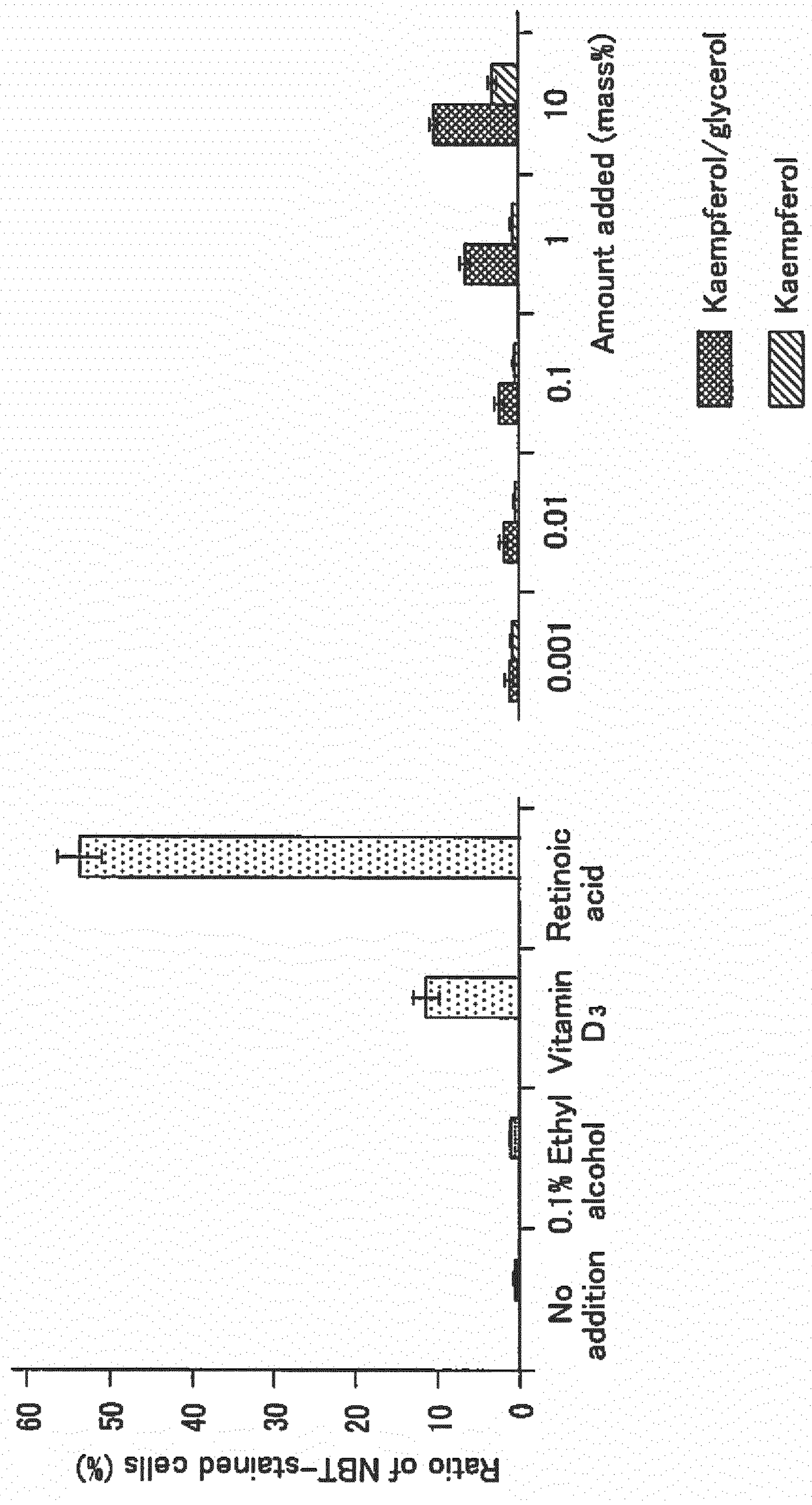

Fig.4

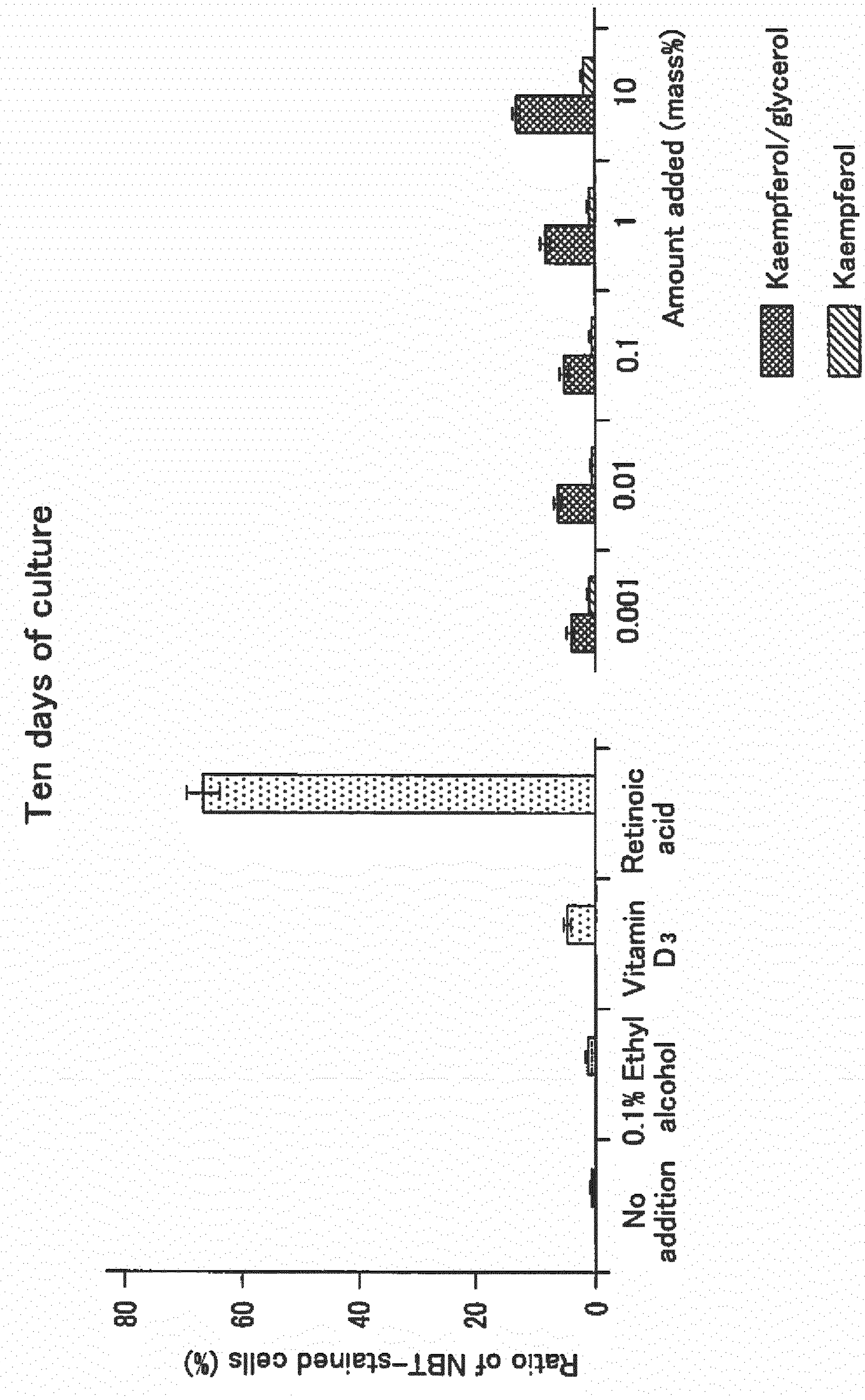
Ten days of culture
Ratio of NBT-stained cells (%)
80
60
40
20
0
No addition
0.1% Ethyl alcohol
Vitamin D3
Retinoic acid
0.001
0.01
0.1
1
10
Amount added (mass%)
Kaempferol/glycerol
Kaempferol

Fig.5

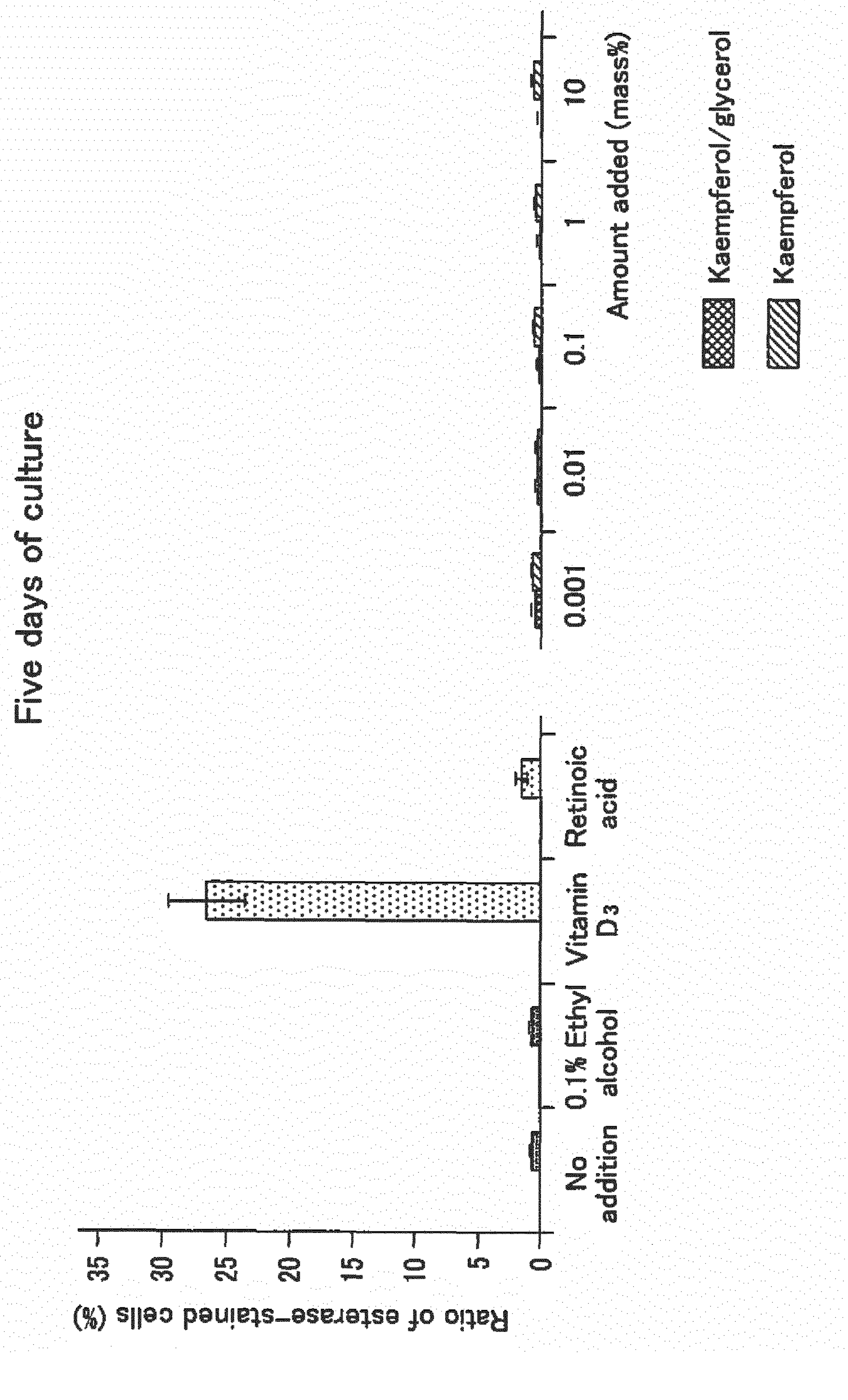
Five days of culture
Ratio of esterase-stained cells (%)
35
30
25
20
15
10
5
0
No addition
0.1% Ethyl alcohol
Vitamin D3
Retinoic acid
0.001
0.01
0.1
1
10
Amount added (mass%)
Kaempferol/glycerol
Kaempferol

Fig.6

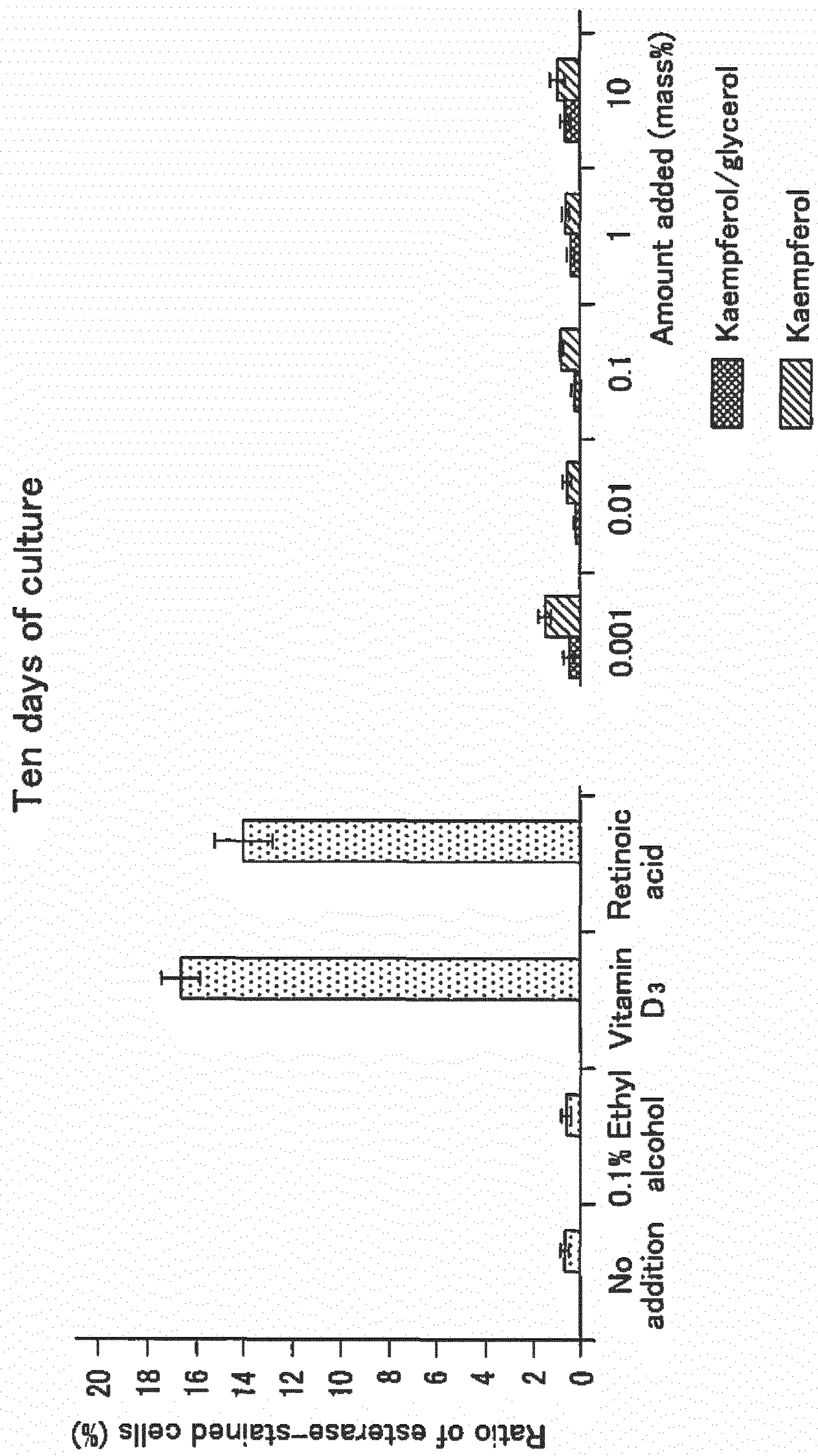
Ten days of culture
Ratio of esterase-stained cells (%)
0
2
4
6
8
10
12
14
16
18
20
No addition
0.1% Ethyl alcohol
Vitamin D3
Retinoic acid
0.001
0.01
0.1
1
10
Amount added (mass%)
Kaempferol/glycerol
Kaempferol

Fig. 7

Ten days of culture

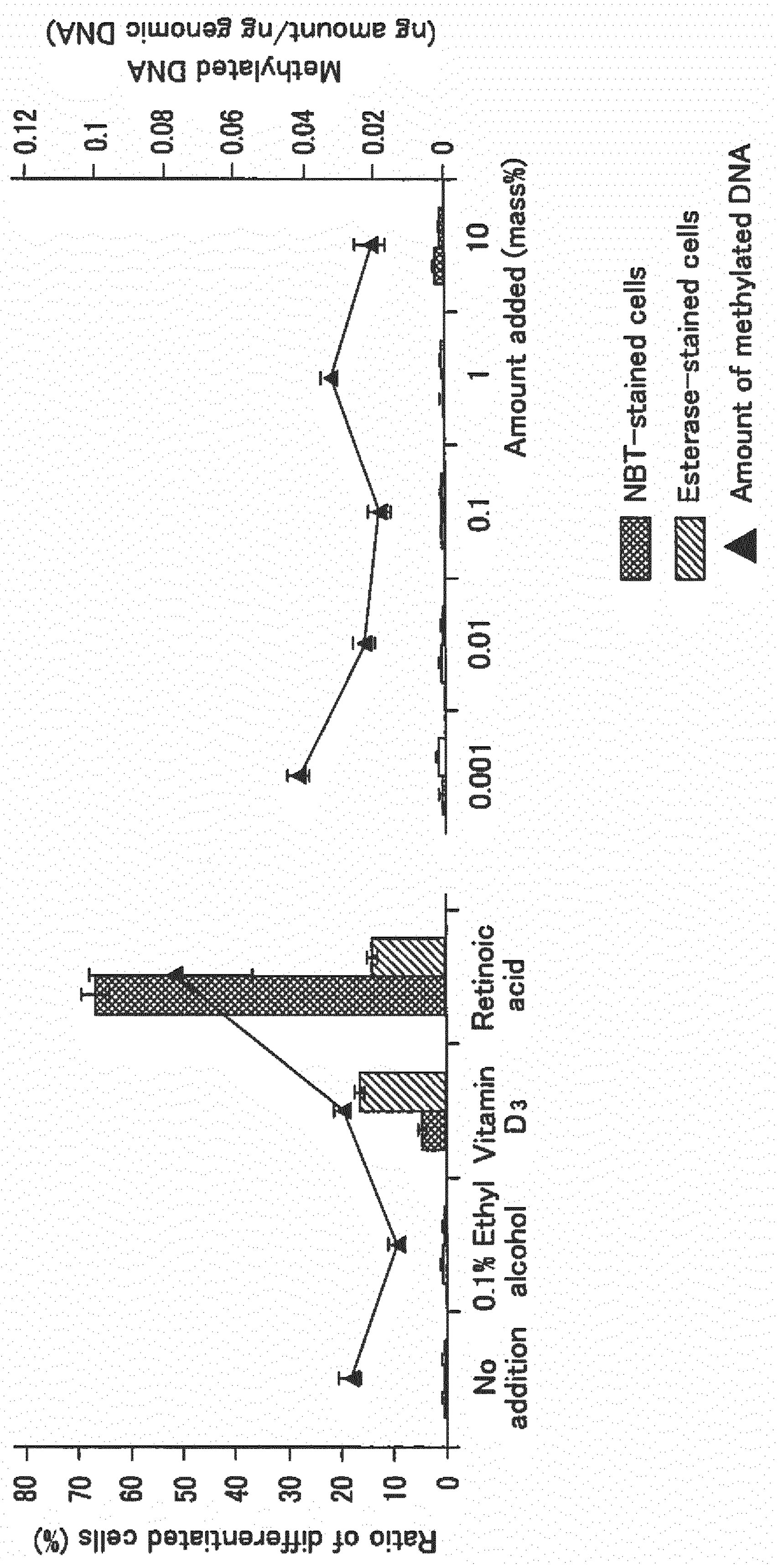

Fig.8

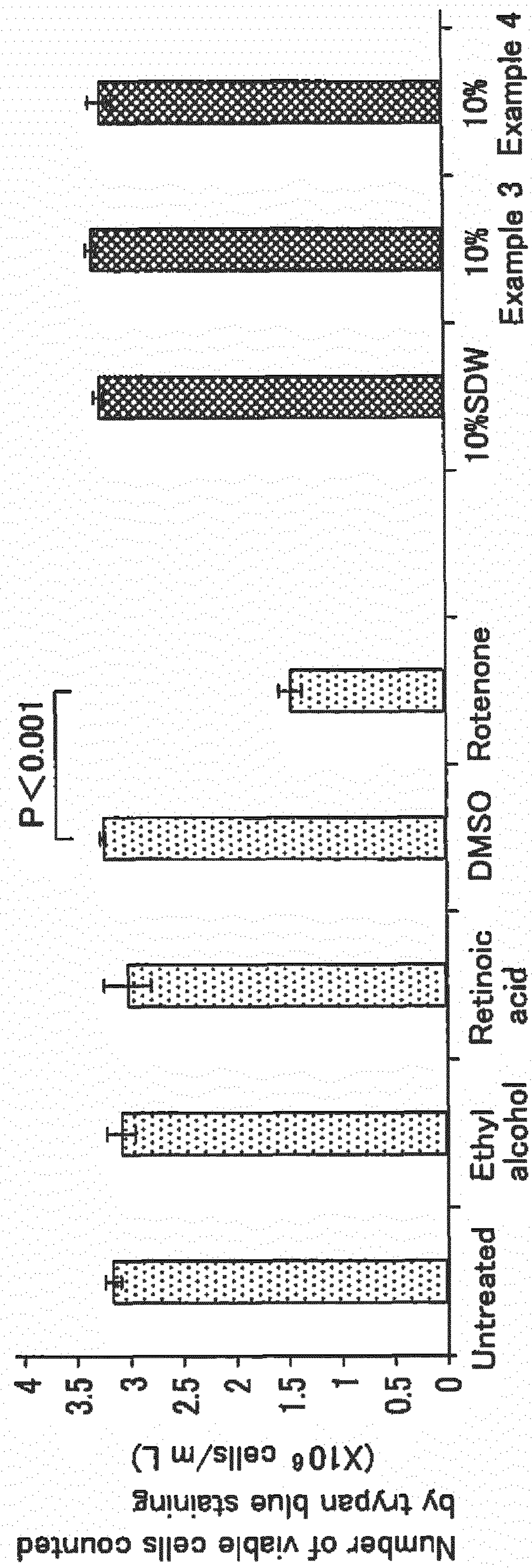
Number of viable cells counted by trypan blue staining (X10⁶ cells/m L)
0
0.5
1
1.5
2
2.5
3
3.5
4
P<0.001
Untreated
Ethyl alcohol
Retinoic acid
DMSO
Rotenone
10%SDW
10% Example 3
10% Example 4

Fig.9

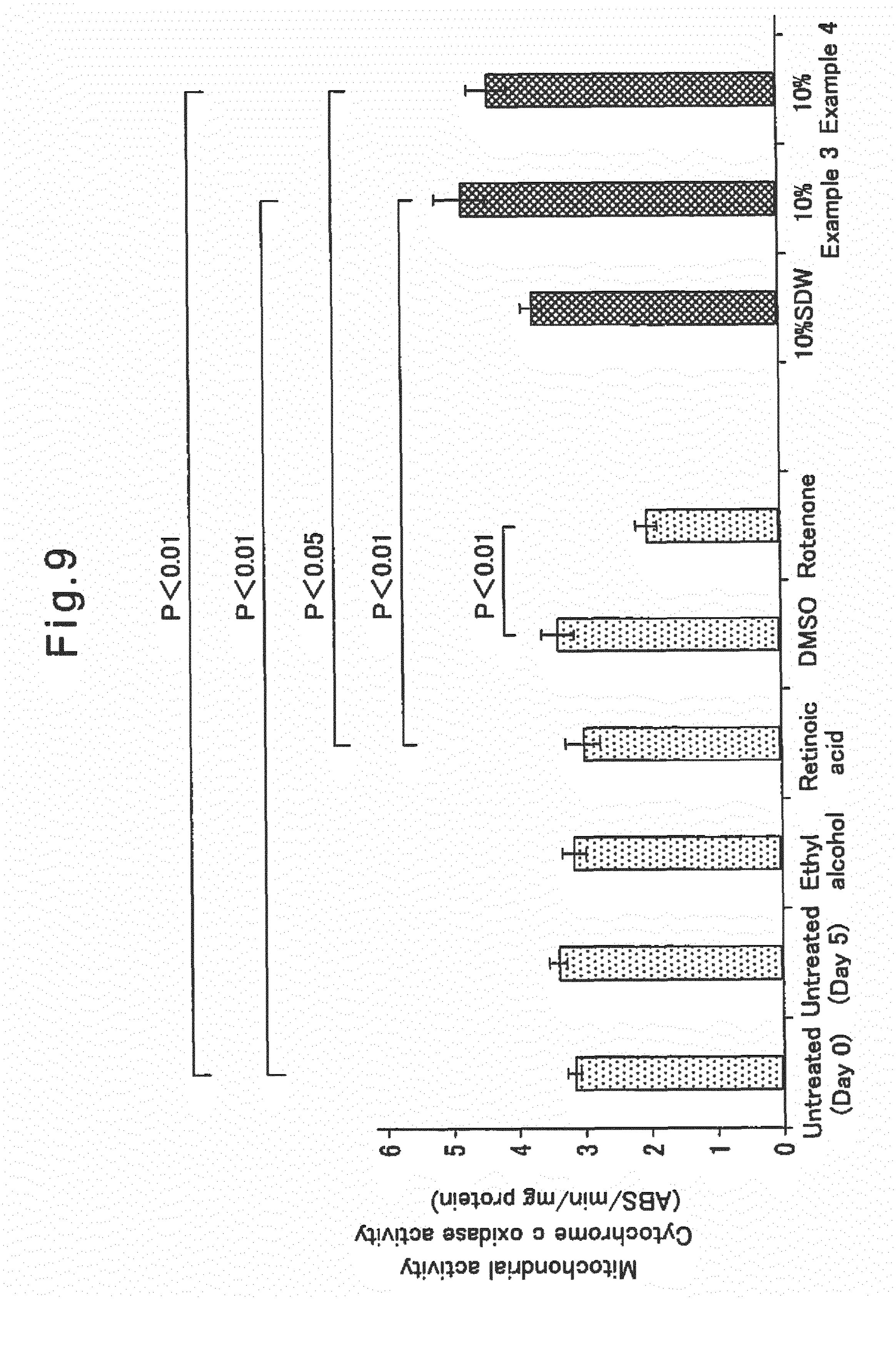
Mitochondrial activity
Cytochrome c oxidase activity
(ABS/min/mg protein)
0
1
2
3
4
5
6
Untreated (Day 0)
Untreated (Day 5)
Ethyl alcohol
Retinoic acid
DMSO
Rotenone
10%SDW
10% Example 3
10% Example 4
P<0.01
P<0.01
P<0.05
P<0.01
P<0.01

Fig.10

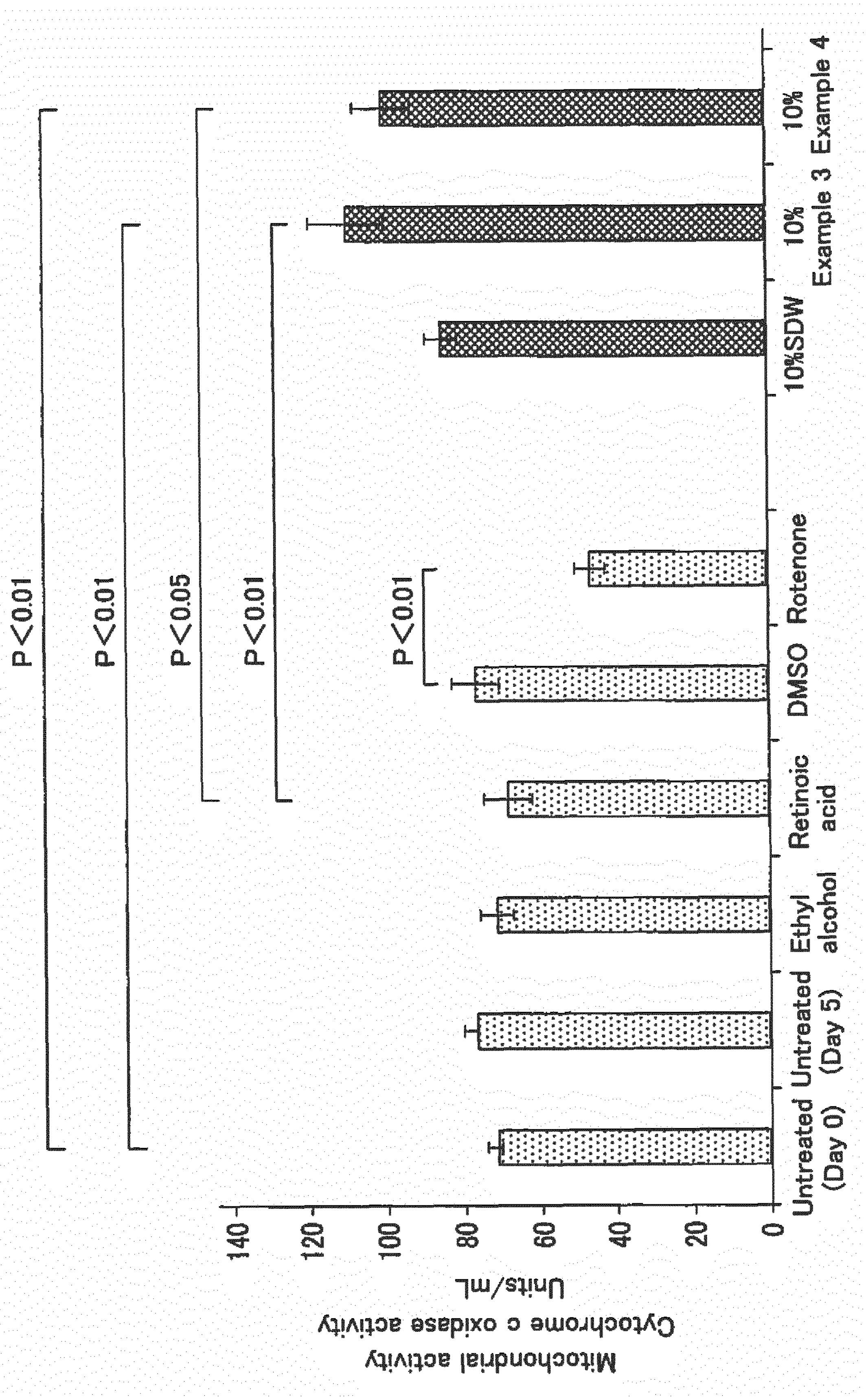
P<0.01
P<0.01
P<0.05
P<0.01
P<0.01
Mitochondrial activity
Cytochrome c oxidase activity
Units/mL
140
120
100
80
60
40
20
0
Untreated (Day 0)
Untreated (Day 5)
Ethyl alcohol
Retinoic acid
DMSO
Rotenone
10%SDW
10% Example 3
10% Example 4

**INTERNATIONAL SEARCH REPORT**

International application No.
PCT/JP2014/059739

A. CLASSIFICATION OF SUBJECT MATTER
*A61K31/352*(2006.01)i, *A61K47/10*(2006.01)i, *A61P35/02*(2006.01)i, *A61P43/00* (2006.01)i

According to International Patent Classification (IPC) or to both national classification and IPC

B. FIELDS SEARCHED

Minimum documentation searched (classification system followed by classification symbols)
A61K31/33-33/44, A61K47/10, A61P35/02, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY/MEDLINE/EMBASE/BIOSIS(STN), JSTPlus/JMEDPlus/JST7580 (JDreamIII), WPI, Science Direct, CiNii, Ichushi WEB

C. DOCUMENTS CONSIDERED TO BE RELEVANT

| Category* | Citation of document, with indication, where appropriate, of the relevant passages | Relevant to claim No. |
|---|---|---|
| X<br>Y | Eun Mi CHOI, Kaempferol protects MC3T3-E1 cells through antioxidant effect and regulation of mitochondrial function, Food and Chemical Toxicology, 2011.05.05, Vol.49, pp.1800-1805 | 1<br>2-5 |
| X<br>Y | Mayte MONTERO et al., Direct activation of the mitochondrial calcium uniporter by natural plant flavonoids, Biochemical Journal, 2004. 11.15, Vol.384, Part 1, pp.19-24 | 1<br>2-5 |
| Y | JP 2011-503211 A (The General Hospital Corp.), 27 January 2011 (27.01.2011), claims 1, 12 to 14; paragraph [0049] & US 2011/0091387 A1 & WO 2009/065141 A1 & CN 101918037 A | 2-5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 April, 2014 (24.04.14) | 13 May, 2014 (13.05.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2014/059739

C (Continuation). DOCUMENTS CONSIDERED TO BE RELEVANT

| Category* | Citation of document, with indication, where appropriate, of the relevant passages | Relevant to claim No. |
|---|---|---|
| Y | Charles S. BESTWICK et al., The effect of short-term kaempferol exposure on reactive oxygen levels and integrity of human(HL-60) leukaemic cells, Biochimica et Biophysica Acta, 2004.11.03, Vol.1740, pp.340-349 | 4,5 |
| A | JP 2012-25724 A (Acteiive Corp.), 09 February 2012 (09.02.2012), claims 1 to 11 (Family: none) | 1-5 |
| A | JP 2005-247753 A (Flora Co., Ltd.), 15 September 2005 (15.09.2005), claims 1 to 4 (Family: none) | 1-5 |
| A | WO 2012/061958 A1 (Tianjin Medical University), 18 May 2012 (18.05.2012), claims & US 2013/0231492 A1 & CN 103209971 A | 1-5 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004067629 A
- JP 2012025724 A
- JP 2007284369 A
- JP 4717362 B